(19) Europäisches Patentamt
European Patent Office
Office européen des brevets

(11) Veröffentlichungsnummer: **0 103 830 A2**

(12) **EUROPÄISCHE PATENTANMELDUNG**

(21) Anmeldenummer: 83108981.8

(22) Anmeldetag: 12.09.83

(51) Int. Cl.³: C 07 C 91/16
C 07 C 91/30, C 07 C 93/00
C 07 C 93/14, C 07 C 103/29
C 07 C 149/36, C 07 C 149/42
A 61 K 31/135, A 61 K 31/165
A 61 K 31/215, A 23 K 1/16

(30) Priorität: 22.09.82 DE 3234995
22.02.83 DE 3306159

(43) Veröffentlichungstag der Anmeldung:
28.03.84 Patentblatt 84/13

(84) Benannte Vertragsstaaten:
AT BE CH DE FR GB IT LI LU NL SE

(71) Anmelder: BAYER AG
Konzernverwaltung RP Patentabteilung
D-5090 Leverkusen 1 Bayerwerk(DE)

(72) Erfinder: Böshagen, Horst, Dr.
Wiesenstrasse 4
D-5657 Haan(DE)

(72) Erfinder: Stoltefuss, Jürgen, Dipl.-Ing.
Parkstrasse 20
D-5657 Haan(DE)

(72) Erfinder: Berschauer, Friedrich, Dr.
Claudiusweg 9
D-5600 Wuppertal(DE)

(72) Erfinder: De Jong, Anno, Dr.
Stockmannsmühle 46
D-5600 Wuppertal 1(DE)

(72) Erfinder: Scheer, Martin, Dr.
Herberts-Katernberg 7
D-5600 Wuppertal 1(DE)

(72) Erfinder: Horstmann, Harald, Dr.
Claudiusweg 19
D-5600 Wuppertal 1(DE)

(72) Erfinder: Seidle, Peter-Rudolf, Dr.
Alte Heide 5d
D-5000 Köln 90(DE)

(54) **Wachstumsfördernde Phenylethylamin-Derivate.**

(57) Die Erfindung betrifft Phenylethylamin-Derivate der allgemeinen Formel I

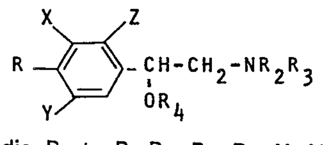

(I)

in der die Reste R, $R_2$, $R_3$, $R_4$, X, Y und Z die in der Beschreibung angegebene Bedeutung haben und deren Verwendung als wachstumsfördernde Zusätze in der Tiernahrung.

EP 0 103 830 A2

BAYER AKTIENGESELLSCHAFT 5090 Leverkusen, Bayerwerk

Zentralbereich
Patente, Marken und Lizenzen Ad/bc/c

Wachstumsfördernde Phenylethylamin-Derivate

Die Erfindung betrifft Phenylethylamin-Derivate und deren Verwendung als wachstumsfördernde Zusätze in der Tiernahrung.

Die Verwendung von Futtermittelzusätzen zur Erzielung höherer Gewichtszuwächse und verbesserter Futterausnutzung wird in der Tierernährung insbesondere bei der Mast von Schweinen, Rindern und Geflügel bereits weitgehend praktiziert.

Es wurde nun gefunden, daß Phenylethylamin-Derivate der allgemeinen Formel (I)

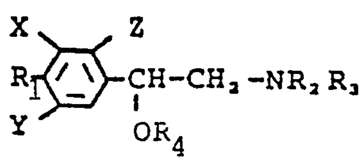

(I)

in der

X,Y und Z gleich oder verschieden sind und Wasserstoff, Halogen, Alkyl, Alkoxy, Alkenyl, Hydroxy, Cyano oder eine COR'-Gruppe bedeuten, und

$R_1$ für Wasserstoff, Alkyl, Halogen, Hydroxy, Alkoxy, Alkylthio, substituiertes oder unsubstituiertes Aryl, substituiertes oder unsubstituiertes Aryloxy oder Arylthio, oder für Acyloxy oder Silyloxy steht, R' für Hydroxy, Alkoxy oder Amino steht, und

$R_2$,$R_3$ gleich oder verschieden sein können und für Wasserstoff, geradkettige oder verzweigte, gegebenenfalls durch Halogen oder Phenyl substituierte $C_1$-$C_8$-Alkyl-, $C_2$-$C_4$-Alkenyl- und Alkinyl-, $C_1$-$C_8$-Hydroxyalkyl-, Alkoxyalkyl-, Aminoalkyl-, Cycloalkyl- oder Aralkyl-Reste, für einen aliphatischen oder aromatischen Acylrest oder einen Silylrest oder für substituiertes oder unsubstituiertes Aryl, wobei bevorzugte Substituenten des Arylrestes beispielsweise Halogen, Alkyl, Alkoxy, Halogenalkyl, Halogenalkoxy sowie substituiertes oder unsubstituiertes Aryloxy oder Arylthio sind, oder für bestimmte Heterocyclen wie für Imidazolinyl, Thiazolinyl oder Pyrimidyl stehen, oder zusammen mit dem Stickstoffatom einen gegebenenfalls substituierten Pyrimidin-, Piperidin-, Piperazin-, Morpholin- oder einen gesättigten bicyclischen Rest bilden, und

$R_4$ für Wasserstoff, einen aliphatischen oder aromatischen Acylrest oder für einen Silylrest steht,

und Phenylethylamin-Derivate der allgemeinen Formel II

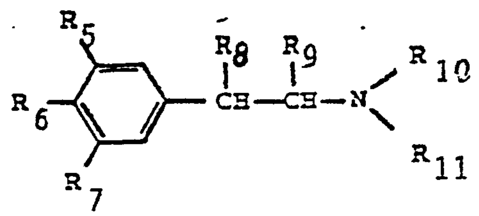

(II)

in welcher

$R_5$, $R_6$ und $R_7$ gleich oder verschieden sind und jeweils für Wasserstoff, Hydroxy, Alkoxy oder Hydroxymethyl stehen, und

$R_8$ Wasserstoff oder Hydroxy bedeutet und

$R_9$ für Wasserstoff, geradkettiges, verzweigtes oder cyclisches Alkyl oder Alkenyl, Aryl, Acyl oder Aroyl steht, wobei die genannten Alkyl-, Alkenyl- und Arylreste gegebenenfalls substituiert sind durch Halogen, Hydroxy, Alkyl, Alkoxy, Amino, gegebenenfalls substituiertes Phenyl oder Heteroaryl, und

$R_{10}$ und $R_{11}$ gleich oder verschieden sind und jeweils für Wasserstoff, geradkettiges, verzweigtes oder cyclisches Alkyl, Alkenyl, Aryl, Acyl, Aroyl, Mono- und Dialkylaminoalkyl, Alkoxyalkyl, Phenoxyalkyl oder Acylamino stehen, wobei die genannten Alkyl-, Alkenyl- und

Arylreste gegebenenfalls substituiert sind durch Halogen, Amino, Alkyl, Alkoxy, Hydroxy, Acylamino, gegebenenfalls substituiertes Phenyl oder Heteroaryl, oder

$R_{10}$ und $R_{11}$ gemeinsam mit dem Stickstoffatom einen gegebenenfalls substituierten Pyrrolidin-, Piperidin-, Piperazin- oder Morpholin-Rest bilden,

und deren physiologisch verträglichen Salze eine ausgezeichnete wachstumsfördernde Wirkung besitzen.

Herstellungsverfahren für Verbindungen dieser Art sind bekannt und werden zum Beispiel beschrieben in (a) A. Kleemann, Pharmazeutische Wirkstoffe: Synthesen, Patente, Anwendungen; S. 35, 62, 169, 174, 175, 190, 196, 254, 296, 338, 346, 387, 389, 427, 438, 461, G. Thieme, Stuttgart 1978; und in (b) O. Schier und A. Marxer in: Ullmanns Encyklopädie der technischen Chemie, Band 12, S. 647-663, Verlag Chemie, Weinheim-New York 1976, (4. Aufl.).

Soweit die Verbindungen der Formel II bekannt sind, handelt es sich bei ihnen um Sympathomimetika mit direktem oder indirektem Angriff auf α- und/oder ßadrenerge Rezeptoren. Sie zeigen in bekannter Weise ausgeprägte Kreislauf- und Gefäßwirkungen sowie Wirkungen auf den Respirationstrakt. Die Wirkungen werden zum Beispiel beschrieben in (a) G. Ehrhart und H. Ruschig, Arzneimittel, Band 2, S. 133-165 und S. 257-269, Verlag

Chemie, Weinheim 1972 (2. Aufl.); (b) G. Ehrhart und H. Ruschig, Arzneimittel, Band 3, S. 63-68, Verlag Chemie, Weinheim 1972 (2. Aufl.); (c) E. Mutschler, Arzneimittelwirkungen, S. 242-265, Wissenschaftliche Verlagsgesellschaft, Stuttgart 1981 (4. Aufl.) und (d) Progress in Medicinal Chemistry, Vol. 6, S. 200-265, Edit.: G.P. Ellis und G.B. West, Butterworths London 1969.

Bevorzugte Verbindungen der Formel I sind Phenylethylamin-Derivate, in denen

X,Y und Z gleich oder verschieden sein können und Wasserstoff, Alkyl, Halogen, Hydroxy oder Alkoxy bedeuten,

$R_1$ Wasserstoff, Hydroxy, $C_1$-$C_4$-Alkoxy oder substituiertes oder unsubstituiertes $C_6$-$C_{10}$-Aryloxy oder Arylthio bedeutet, und

$R_2$,$R_3$ gleich oder verschieden sein können und für Wasserstoff, gegebenenfalls durch Halogen, Hydroxy oder Phenyl substituierte niedere Alkylreste oder für gegebenenfalls durch Halogen, $C_1$-$C_4$-Alkyl-, $C_1$-$C_4$-Alkoxy-, $C_6$-$C_{10}$-Aryloxy- oder Arylthio-substituierte Phenylreste stehen und

$R_4$ für Wasserstoff steht.

Besonders bevorzugte Verbindungen sind Phenylethylamin-Derivate der Formel (I), in der

X,Y und Z gleich oder verschieden sein können und für Wasserstoff oder Halogen stehen,

$R_1$ für Hydroxy oder $C_1$-$C_4$-Alkoxy steht und

$R_2,R_3$ gleich oder verschieden sein können und Wasserstoff, $C_1$-$C_4$-Alkylreste oder durch gegebenenfalls substituiertes $C_6$-$C_{10}$-Aryloxy substituiertes Phenyl bedeuten, und

$R_4$ Wasserstoff bedeutet.

Die erfindungsgemäß verwendeten Phenylethylamin-Derivate der Formel I sind in der Literatur nicht beschrieben, können aber nach bekannten Verfahren hergestellt werden, indem man

a) Verbindungen der Formel (III)

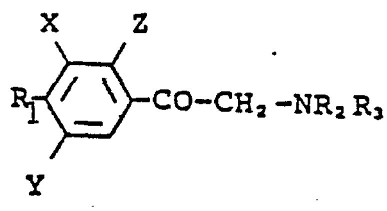

(III)

in der

X,Y,Z,$R_1$,$R_2$ und $R_3$ die oben angegebene Bedeutung haben,

reduziert oder indem man

b) Verbindungen der Formel (IV)

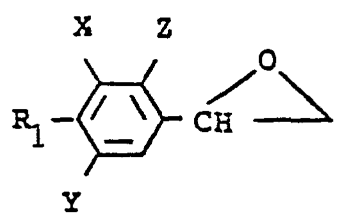

(IV)

in der

X,Y,Z und $R_1$ die oben angegebene Bedeutung haben,

mit Verbindungen der Formel (V)

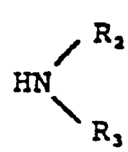

(V)

in der
$R_2$ und $R_3$ die oben angegebenen Bedeutung haben, umsetzt.

Verbindungen der Formel I, in denen $R_1$ Acyloxy oder Silyloxy ist und/oder $R_2$ bzw. $R_3$ und/oder $R_4$ für einen aliphatischen oder aromatischen Acyl- bzw. Silylrest stehen, werden nach bekannten Methoden aus Verbindungen der allgemeinen Formel I, in der R Hydroxy und/oder $R_2$ bzw. $R_3$ und/oder $R_4$ Wasserstoff bedeuten, durch Umsetzung mit geeigneten Acylierungs- bzw. Silylierungsmitteln erhalten.

Die hier verwendeten Ausgangsstoffe (III) und (IV) sind entweder bekannt oder können nach bekannten Herstellungsverfahren dargestellt werden (vgl. Lutz et al., J.Org. Chem. 12, 617-703, (1947); G.C. King und G.K. Ostrum, J.Org.Chem. 29, 3459 (1964)).

Bevorzugt eignen sich für den erfindungsgemäßen Verwendungszweck Phenylethylamin-Derivate der Formel II, in der

$R_5$, $R_6$ und $R_7$ gleich oder verschieden sind und jeweils für Wasserstoff, Hydroxy, Hydroxymethyl oder Methoxy stehen,

$R_8$ Wasserstoff oder Hydroxy bedeutet,

$R_9$ für Wasserstoff, geradkettige oder verzweigte Alkyl- oder Alkenyl-Gruppen mit bis zu 5 Kohlenstoffatomen, Phenyl oder Aralkyl mit 7 bis 10 Kohlenstoffatomen steht, wobei die genannten Aryl-Reste

gegebenenfalls substituiert sind durch Chlor, Fluor, niederes Alkyl mit bis zu 4 Kohlenstoffatomen, Hydroxy oder Alkoxy, und

$R_{10}$ und $R_{11}$ gleich oder verschieden sein können und für Wasserstoff, geradkettiges oder verzweigtes Alkyl mit 1 bis 8 Kohlenstoffatomen, Alkenyl mit 2 bis 4 Kohlenstoffatomen, Hydroxyalkyl, Mono- und Dialkylaminoalkyl und Phenylalkylaminoalkyl mit jeweils bis zu 4 Kohlenstoffatomen in den Alkylresten, oder Phenyl-, Aralkyl-, Methylendioxyphenylalkyl-, Alkoxyalkyl-Reste mit 8 bis 12 Kohlenstoffatomen oder Phenoxyalkylreste mit 8 bis 16 Kohlenstoffatomen, wobei die genannten Alkylgruppen gegebenenfalls durch Methyl oder Ethyl und die Phenylreste gegebenenfalls durch Halogen, insbesondere durch Chlor oder Fluor, Hydroxy, niederes Alkyl oder niederes Alkoxy mit bis zu 2 Kohlenstoffatomen substituiert sein kann, stehen, oder

$R_{10}$ und $R_{11}$ gemeinsam mit dem Stickstoffatom einen gegebenenfalls substituierten Pyrrolidin-, Piperidin-, Piperazin- oder Morpholin-Rest bilden.

Von ganz besonderer Bedeutung sind Verbindungen der allgemeinen Formel II, in welcher

$R_5$, $R_6$ und $R_7$ gleich oder verschieden sind und jeweils für Wasserstoff oder Hydroxy stehen und

$R_8$ Hydroxy bedeutet, und

$R_9$ für Wasserstoff oder Methyl steht und

$R_{10}$ und $R_{11}$ gleich oder verschieden sind und jeweils für Wasserstoff, geradkettiges oder verzweigtes Alkyl mit jeweils bis zu 4 Kohlenstoffatomen stehen, wobei die Alkylgruppen gegebenenfalls durch Phenyl, Phenoxy, Hydroxyphenyl oder Methylendioxyphenyl substituiert sein können.

Die Wirkstoffe können in allen Bereichen der Tierzucht als Mittel zur Förderung und Beschleunigung des Wachstums und zur Verbesserung der Futterverwertung bei gesunden und kranken Tieren verwendet werden.

Die Wirksamkeit der Wirkstoffe ist hierbei weitgehend unabhängig von der Art und dem Geschlecht der Tiere. Besonders wertvoll erweisen sich die Wirkstoffe bei der Aufzucht und Haltung von Jung- und Masttieren. Als Tiere, bei denen die Wirkstoffe zur Förderung und Beschleunigung des Wachstums und zur Verbesserung der Futterverwertung eingesetzt werden können, seien beispielsweise folgende Nutz- und Ziertiere genannt:

Warmblüter wie Rinder, Schweine, Pferde, Schafe, Ziegen, Katzen, Hunde, Kaninchen, Pelztiere, z.B. Nerze, und Chinchilla, Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50, insbesondere 0,1 bis 10 mg/kg Körpergewicht pro Tag. Die Dauer der Verabreichung kann von wenigen Stunden oder Tagen bis zu mehreren Jahren betragen. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen. Aus Zweckmäßigkeitsgründen ist in den meisten Fällen eine orale Verabreichung, insbesondere im Rhythmus der Nahrungs- und/oder Getränkeaufnahme der Tiere, vorzuziehen.

Die Wirkstoffe können als reine Stoffmischung oder in formulierter Form, also in Mischung mit nichttoxischen

inerten Trägerstoffen beliebiger Art, z.B. mit Trägerstoffen und in Formulierungen, wie sie bei nutritiven Zubereitungen üblich sind, verabreicht werden.

Die Wirkstoffe werden gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter und/oder Trinkwasser, wobei je nach Bedarf der Wirkstoff der Gesamtmenge oder nur Teilen des Futters und/oder des Trinkwassers zugegeben wird.

Die Wirkstoffe werden nach üblichen Methoden durch einfaches Mischen als reine Stoffmischung, vorzugsweise in feinverteilter Form oder in formulierter Form in Mischung mit eßbaren nichttoxischen Trägerstoffen, gegebenenfalls in Form eines Praemix oder eines Futterkonzentrates, dem Futter und/oder Trinkwasser beigefügt.

Das Futter und/oder Trinkwasser kann beispielsweise die Wirkstoffe in einer Gewichtskonzentration von etwa 0,01 bis 50, insbesondere 0,1 bis 10 ppm, enthalten. Die optimale Höhe der Konzentration der Wirkstoffe in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder

speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten die Wirkstoffe neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentraten können die Wirkstoffe gegebenenfalls auch durch ihre Oberfläche bedeckende geeignete Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das einen erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung

und 2,5 g Wirkstoff-Praemix ergeben nach sorgfältigem Mischen 1 kg Futter.

In einem kg Futtermischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$.

Der Wirkstoff-Praemix enthält die Wirkstoffe in der gewünschten Menge, z.B. 10 mg und zusätzlich 1 g DL-Methionin sowie so viel Sojabohnenmehl, daß 2,5 g Praemix entstehen.

Beispiel für die Zusammensetzung eines Schweineaufzuchtfutters, das einen erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auf zur Aufzucht und Mast anderer Tiere verwendet werden.

Mit den erfindungsgemäßen Wirkstoffen wurden mehrere Fütterungs- und Stoffwechseluntersuchungen durchgeführt. Die verwendeten Wirkstoffe sind die der Herstellungsbeispiele 1 und 2.

Dabei wurden folgende Ergebnisse erhalten:

Beispiel 1

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | |

Rohnährstoffe *

| | |
|---|---|
| Rohprotein | 19,0 |
| Rohfett | 4,0 |
| Rohfaser | 6,0 |
| Asche | 7,0 |
| Wasser | 13,5 |
| N-freie Extraktst. | 50,5 |

Umsetzbare Energie:

| | |
|---|---|
| Kcal/kg | 3100 |
| KJ/kg | 13000 |

Mineralstoffe *

| | |
|---|---|
| Calcium | 0,9 |
| Phosphor | 0,7 |
| Magnesium | 0,2 |
| Natrium | 0,2 |

Vitamine **

Standard-Diät

| | |
|---|---|
| Vitamin A | 15000 IE |
| Vitamin $D_3$ | 600 IE |
| Vitamin E | 75 mg |
| Vitamin $K_3$ | 3 mg |
| Vitamin $B_1$ | 18 mg |
| Vitamin $B_2$ | 12 mg |
| Vitamin $B_6$ | 9 mg |
| Vitamin $B_{12}$ | 24 mcg |
| Nikotinsäure | 36 mg |
| Pantothensäure | 21 mg |
| Folsäure | 2 mg |
| Biotin | 60 mg |
| Cholin | 600 mg |
| Vitamin C | 36 mg |

Aminosäuren *

| | |
|---|---|
| Lysin | 0,9 |
| Methionin + Cystin | 0,6 |
| Phenylalanin+Tyrosin | 1,4 |
| Arginin | 1,1 |
| Histidin | 0,4 |
| Tryptophan | 0,2 |
| Threonin | 0,6 |
| Isoleucin | 0,9 |
| Leucin | 1,3 |
| Valin | 0,9 |

Spurenelemente **

| | |
|---|---|
| Magnan | 75,0 |
| Eisen | 135,0 |
| Kupfer | 13,0 |
| Zink | 70,0 |
| Jod | 0,9 |
| Fluor | 9,0 |

* % in der Diät (Mittelwert)
** mg in 1 kg Diät (Mittelwert)

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)

b 2) 25 ppm (Wirkstoff Herstellungsbeispiel 1) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 153 | 27,9 | 5,62 |
| c 2) 25 ppm (Wirkstoff Herstellungsbeispiel 1) | 175 | 43,7 | 4,03 |

Beispiel 2

a) Tier-Charakteristik und Futter

| | |
|---|---|
| a 1) Ratten, weiblich | |
| a 2) Anzahl | 30 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter

ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 7-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 24)
b 2) 0,2 ppm (Wirkstoff Herstellungsbeispiel 1)

c) Ergebnis (Futteraufnahme, Wachstum, Futterverwertung) während der gesamten Versuchsperiode (12 Tage)

| | Futter-aufnahme (g) | Zuwachs (g) | Futter-verwer-tung (g/g) |
|---|---|---|---|
| c 1) Negativkontrolle | 200 | 41,0 | 4,88 |
| c 2) 0,2 ppm (Wirkstoff Herstellungsbeispiel 1) | 213 | 50,5 | 4,21 |

Beispiel 3

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Herstellungsbeispiel 1) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 182 | 37,0 | 5,04 |
| c 2) 25 ppm (Wirkstoff Herstellungsbeispiel 1) | 187 | 42,4 | 4,41 |

Beispiel 4

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den

Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Herstellungsbeispiel 2) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 196 | 41,3 | 4,75 |
| c 2) 25 ppm (Wirkstoff Herstellungsbeispiel 2) | 199 | 45,1 | 4,41 |

Beispiel 5

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Herstellungsbeispiel 2) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 152 | 27,9 | 5,62 |
| c 2) 25 ppm (Wirkstoff Herstellungsbeispiel 2) | 171 | 43,4 | 3,96 |

Beispiel 6

a) Tier-Charakteristik und Futter

| | |
|---|---|
| a 1) Ratten, weiblich | |
| a 2) Anzahl | 18 |
| a 3) Zucht | SPF Wistar, Züchtung Hagemann |
| a 4) Gewicht | 90-150 g |
| a 5) Zustand | gut |
| a 6) Futter | wie in Beispiel 1 |

b) Behandlung der Tiere

Die Tiere wurden 2 Tage an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren. Nach einer 5-tägigen Vorperiode wurde eine 8-tägige Hauptperiode durchgeführt, in denen Futteraufnahme, Zuwachs und Futterverwertung bestimmt wurden.

Es wurden folgende Behandlungen geprüft:

b 1) Negativkontrolle (n = 12)
b 2) 25 ppm (Wirkstoff Herstellungsbeispiel 2) (n = 6)

c) Ergebnis (Futteraufnahme, Wachstum, Futterverwertung) während der gesamten Versuchsperiode (13 Tage)

| | Futteraufnahme (g) | Zuwachs (g) | Futterverwertung (g/g) |
|---|---|---|---|
| c 1) Negativkontrolle | 182 | 37,0 | 5,04 |
| c 2) 25 ppm (Wirkstoff Herstellungsbeispiel 2) | 183 | 43,2 | 4,36 |

Beispiel 7 - Ratten

a. Versuchsbeschreibung

Vor Beginn des Fütterungsversuches wurden die Ratten zwei Tage lang an die neuen Haltungsbedingungen adaptiert, wobei generell das Versuchsfutter ohne Wirkstoffzusatz verabreicht wurde. Am dritten Versuchstag wurden die Tiere randomisiert und anschließend die Versuchsgruppen so gebildet, daß sowohl die Mittelwerte als auch die Streuungen in den Körpergewichten zwischen den Gruppen gleich waren.

Der Fütterungsversuch, in dem die Futteraufnahme, der Körpergewichtszuwachs und die Futterverwertung individuell bestimmt wurden, erstreckte sich über 15 Tage.

Als Wirkstoffe wurden dem Futter untergemischt:

1-(3,4-Dihydroxyphenyl)-2-/1-methyl-2-(3,4-methylendioxyphenyl)ethylamino/ethanolhydrochlorid (= Verbindung I),

2-Amino-1-(3-hydroxyphenyl)ethanolhydrochlorid (= Verbindung II),

2-Amino-1-(3-hydroxyphenyl)propanolhydrogentartrat (= Verbindung III),

1-(4-Hydroxyphenyl)-2-(1-methyl-3-phenylpropylamino)-propanolhydrochlorid (= Verbindung IV),

2-Ethylamino-1-(3-hydroxyphenyl)ethanolhydrochlorid (= Verbindung V),

1-(3,5-Dihydroxyphenyl)-2-(3-phenylpropylamino)propanol als Salz der 4-Aminobenzoesäure (= Verbindung VI),

1-(3-Hydroxyphenyl)-2-(1,1-dimethyl-2-phenylethylamino)-ethanol als Salz der 4-Aminobenzoesäure (= Verbindung VII),

5-/2-/(1,1-Dimethylethyl)amino7-1-hydroxyethyl7-2-hydroxy-benzamid . HCl (= Verbindung VIII)

Die Ergebnisse sind in einer zusammenfassenden Tabelle dargestellt.

b. Tier-Charakteristik und Futter

Ratten, weiblich, SPF-Wistar
36 Tiere/Versuch
Gewicht: 90 bis 150 g
Futter: Haltungsdiät für Ratten mit folgender Zusammensetzung:

| Rohnährstoffe* | | Vitamine** | |
|---|---|---|---|
| Rohprotein | 19,0 | Standard-Diät | |
| Rohfett | 4,0 | Vitamin A | 15.000 IE |
| Rohfaser | 6,0 | Vitamin D, | 1.600 IE |
| Asche | 7,0 | Vitamin E | 75 mg |
| Wasser | 13,5 | Vitamin K, | 3 mg |
| N-freie Extraktst. | 50,5 | Vitamin $B_1$ | 18 mg |
| Umsetzbare Energie: | | Vitamin $B_2$ | 12 mg |
| Kcal/kg | 3.100 | Vitamin $B_6$ | 9 mg |
| KJ/kg | 13.000 | Vitamin $B_{12}$ | 24 mcg |
| | | Nikotinsäure | 36 mg |
| Mineralstoffe* | | Pantothensäure | 21 mg |
| Calcium | 0,9 | Folsäure | 2 mg |
| Phosphor | 0,7 | Biotin | 60 mcg |

| | |
|---|---|
| Magnesium | 0,2 |
| Natrium | 0,2 |

| Cholin | 600 mg |
|---|---|
| Vitamin C | 36 mg |

Spurenelemente**

| | |
|---|---|
| Mangan | 75,0 |
| Eisen | 135,0 |
| Kupfer | 13,0 |
| Zink | 70,0 |
| Jod | 0,9 |
| Fluor | 9,0 |

Aminosäuren**

| | |
|---|---|
| Lysin | 0,9 |
| Methionin + Cystin | 0,6 |
| Phenylalanin + Tyrosin | 1,4 |
| Arginin | 1,1 |
| Histidin | 0,4 |
| Tryptophan | 0,2 |
| Threonin | 0,6 |
| Isoleucin | 0,9 |
| Leucin | 1,3 |
| Valin | 0,9 |

* in der Diät (Mittelwert)

** mg in 1 kg Diät (Mittelwert)

Beispiel 8 - Broiler

a. Versuchsbeschreibung

Die in Käfigen gehaltenen Tiere wurden im Alter von 3 bis 5 Tagen für den Versuch herangezogen, der sich insgesamt über einen Zeitraum von 14 Tagen erstreckte.

Die Tiere erhielten während dieser Zeit Futter, welchem die beanspruchten Verbindungen
1-(3,4-Dihydroxyphenyl)-2-[1-methyl-2-(3,4-methylendioxyphenyl)-ethylamino]ethanolhydrochlorid (= Verbindung I),

2-Amino-1-(3-hydroxyphenyl)ethanolhydrochlorid (= Verbindung II),

2-Amino-1-(3-hydroxyphenyl)propanolhydrogentartrat (= Verbindung III),

1-(4-Hydroxyphenyl)-2-(1-methyl-3-phenylpropylamino)-propanolhydrochlorid (= Verbindung IV),

2-Ethylamino-1-(3-hydroxyphenyl)ethanolhydrochlorid (= Verbindung V),

1-(3,5-Dihydroxyphenyl-2-(3-phenylpropylamino)propanol als Salz der 4- Aminobenzoesäure (= Verbindung VI),

1-(3-Hydroxyphenyl)-2-(1,1-dimethyl-2-phenylethylamino)-ethanol als Salz der 4-Aminobenzoesäure (= Verbindung VII)
und

5-[2-[(1,1-Dimethylethyl)amino]-1-hydroxyethyl]-2-hydroxybenzamid . HCl (= Verbindung VIII)

in einer Dosis von 5 bzw. 25 ppm beigemischt worden waren. Eine Negativkontrolle (unsupplementiertes Futter) wurde mitgeführt. Bei Versuchsbeginn hatten alle Tiere einer Versuchsgruppe das gleiche Ausgangsgewicht.

Als Beurteilungskriterien wurden die Gewichtszuwachsraten, der Futterverbrauch und die Futterverwertung herangezogen.

Die Ergebnisse sind in einer Tabelle dargestellt.

b. Tier-Charakteristik und Futter

Sortierte Masthybriden, männlich
24 Tiere/Versuch (4 x 6)
Gewicht 50 bis 65 g
Futter: Höveler Kükenalleinfutter KA 57 ohne Zusätze von Antibiotika und Coccidiostatika folgender Zusammensetzung:

Wertbestimmende Bestandteile:

18 % Rohprotein
7 % Rohfaser
8 % Asche
1 % Kalzium
0,7 % Phosphor

Zusammensetzung:

54 % Futtergetreideschrot (40 % Mais, 12 % Weizen)
17,5 % Sojaschrot



BAD ORIGINAL

5,2 % Maiskleberfutter
5,2 % Weizenvollmehl
3,1 % Fischmehl
3,1 % Tapiokamehl
3,1 % Luzernegrasgrünmehl
2,1 % Weizenkeime (zerkleinert)
1,7 % Fleischknochenmehl
1,6 % Molkenpulver
1,4 % kohlensaurer Futterkalk
1,0 % phosphorsaurer Futterkalk
1,0 % Melasse

Ergebnisse (Wachstum, Futteraufnahme, Futterverwertung)

a. Ratten

| Substanz | ppm | Zuwachs | Futteraufnahme | Futterverwertung |
|---|---|---|---|---|
| Kontrolle | -- | 100 % | 100 % | 100 % |
| Verbindung I | 25 | 112 % | 104 % | 92,9 % |
| Verbindung II | 25 | 107 % | 98 % | 91,6 % |
| Verbindung III | 25 | 103 % | 99 % | 96,1 % |
| Verbindung IV | 25 | 105 % | 101,7 % | 96,9 % |
| Verbindung V | 25 | 115 | 104 % | 90,4 % |
| Verbindung VIII | 25 | 125,0% | 106 % | 84,8 % |

b. Küken

| Substanz | ppm | Zuwachs | Futteraufnahme | Futterverwertung |
|---|---|---|---|---|
| Kontrolle | -- | 100 % | 100 % | 100 % |
| Verbindung VI | 5 | 101,8 % | 96,2 % | 94,4 % |
| Verbindung V | 5 | 102,0 % | 99,0 % | 97,5 % |
| Verbindung VII | 25 | 102,0 % | 100,0 % | 98,1 % |
| Verbindung II | 25 | 105,0 % | 103,3 % | 98,8 % |

## Herstellungsbeispiele

### Beispiel 1

2-t-Butylamino-1-(4-methoxyphenyl)-ethanol

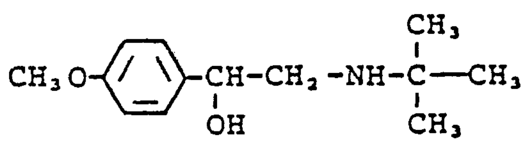

24,1 g (92,2 mmol) ω-tert.-Butylamino-4-methoxy-acetophenon-hydrochlorid werden in 125 ml Methanol und 80 ml Wasser gelöst und tropfenweise mit einer Lösung von 4,25 g (112 mmol) Natriumborhydrid in 8,5 ml Wasser versetzt, wobei die Lösung durch gleichzeitige Zugabe von ca. 15 %iger Salzsäure zwischen pH 3 und pH 7 gehalten wird. Es wird 30 Minuten nachgerührt, mit Salzsäure auf pH 1 gestellt und filtriert. Es wird mit konzentrierter Ammoniaklösung alkalisch gestellt, das ausgefallene Produkt wird abgesaugt, mit Wasser gewaschen und getrocknet. Man erhält 15 g farblose Kristalle vom Schmelzpunkt 104°C.

Analog wurden hergestellt:

| Beispiel Nr. | Strukturformel | Schmelzpunkt °C |
|---|---|---|
| 2 | 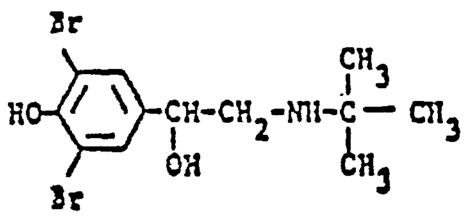 | 190 |
| 3 | 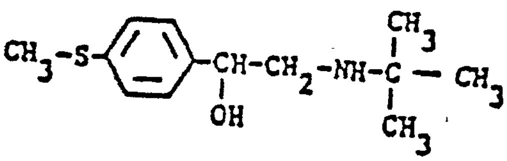 · HCl | 240 |
| 4 | 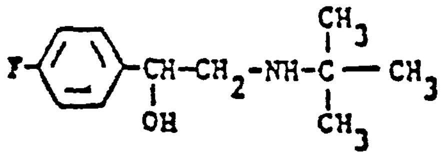 | 96 |
| 5 | 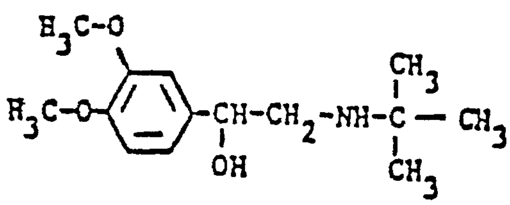 | 107 |

| Beispiel Nr. | Strukturformel | Schmelzpunkt °C |
|---|---|---|
| 6 | 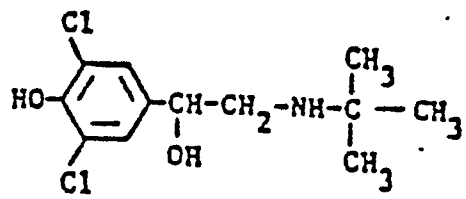 · HCl | 218 |
| 7 | 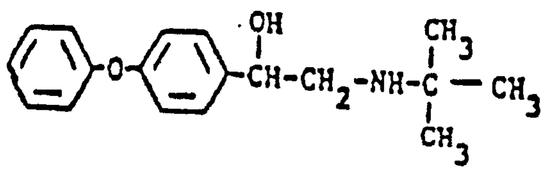 $^1/_2$ 1.5-Naphtalindisulfonsäure | 252 |
| 8 | 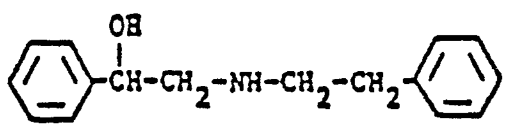 · HCl | 215 |
| 9 | 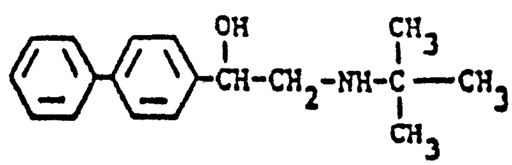 | 139 |
| 10 | 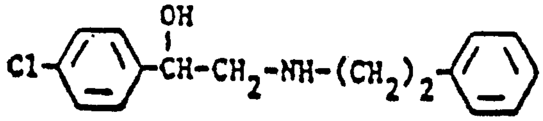 · HCl | 208 |

Analog wurden hergestellt:

| Beispiel Nr. | Strukturformel | Schmelzpunkt °C |
|---|---|---|
| 11 | 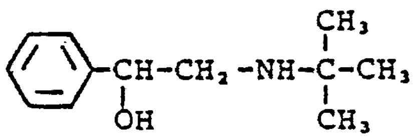 • HCl | 218-220 |
| 12 | 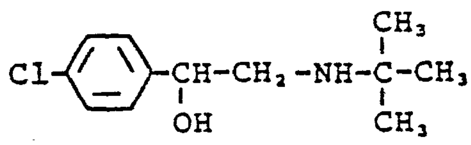 • HCl | 195-197 |
| 13 | 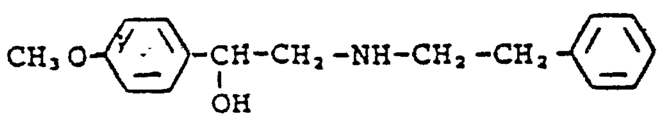 | 107-109 |
| 14 | 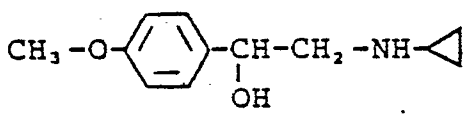 | 75-76 |
| 15 | 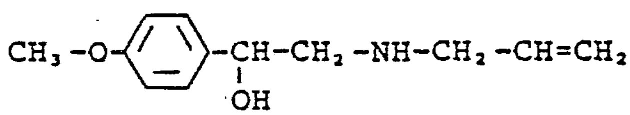 | 82-83 |
| 16 | 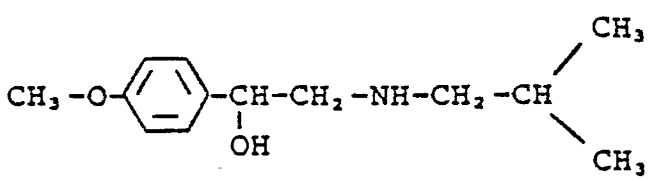 | 82-83 |

Beispiel 17

1-(4-Methoxyphenyl)-2-/3-methyl-4-(4-trifluormethylthio-phenoxy)-phenylamino7-ethanol-1/2-naphthalin-disulfonat

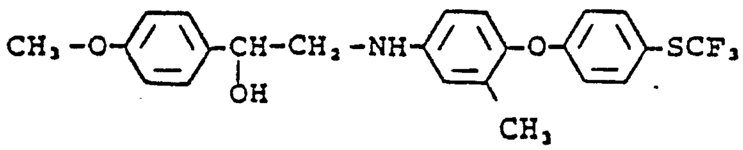 1/2 $C_{10}H_8O_6S_2$

3,1 g 4-Methoxy-ω-/3-methyl-4-(4-trifluormethylthio-phenyl)-phenylamino7-acetophenon werden in einer Mischung von 20 ml absolutem Tetrahydrofuran und 100 ml Methanol gelöst und unter Rühren mit 0,3 g Natriumborhydrid versetzt. Es wird 1 h gerührt, mit 1 N Salzsäure sauer gestellt und eingeengt. Der Eindampfrückstand wird mit Essigester/Natriumhydrogencarbonatlösung aufgenommen und getrennt. Die Essigesterphase wird 2 mal mit Wasser gewaschen, getrocknet und eingeengt. Da das Produkt nicht kristallisiert, wird das Naphthalin-1,5-disulfonat hergestellt. Man erhält 3,4 g des Salzes vom Schmelzpunkt 266-269°C.

Analog wurden erhalten:

Beispiel 18

1-Phenyl-2-/3-methyl-4-(4-trifluormethylthio-phenoxy)-phenylamino7-ethanol, kristallisiert mit 1/2 Naphthalin-1,5-disulfonsäure, Schmelzpunkt 210°C

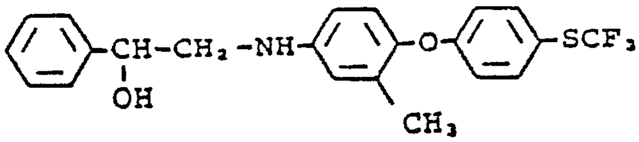

· 1/2

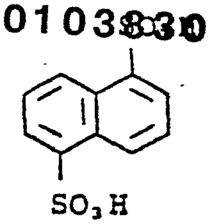

Beispiel 19

2-/4-(4-Chlorphenylthio)-3,5-dimethyl-phenylamino/-1-phenylethanol-hydrochlorid vom Schmelzpunkt 175-180°C unter Zersetzung

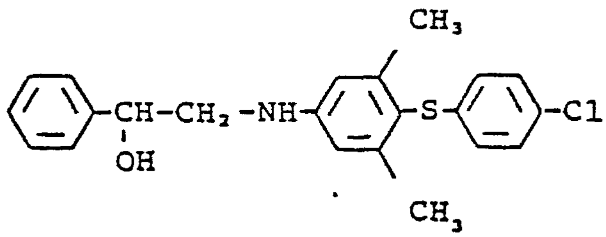

· HCl

Beispiel 20

5-/2-/(1,1-Dimethylethyl)amino/-1-hydroxyethyl/-2-hydroxy-benzamid . HCl

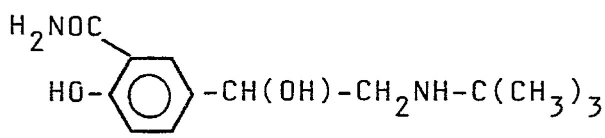

. HCl

6.0 g 5-/2-/N-(1,1-Dimethylethyl)-benzylamino/acetyl/-2-hydroxy-benzamid . HCl werden in einer Mischung aus 120 ml Methanol und 90 ml Wasser gelöst. 1,5 g Pd/C (10 %) werden zugefügt und die Suspension wird bei Raumtemperatur und unter Normaldruck hydriert. Der Katalysator wird entfernt und die Lösung konzentriert. Der Rückstand wird aus Methanol/Isopropylacetat umkristallisiert. Schmelzpunkt 205°C.

## Patentansprüche

1) Phenylethylamin-Derivate der allgemeinen Formel (I)

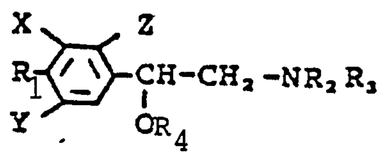

(I)

in der

X,Y und Z gleich oder verschieden sind und Wasserstoff, Halogen, Alkyl, Alkoxy, Alkenyl, Hydroxy, Cyano oder eine COR'-Gruppe bedeuten, und

$R_1$ für Wasserstoff, Alkyl, Halogen, Hydroxy, Alkoxy, Alkylthio, substituiertes oder unsubstituiertes Aryl, substituiertes oder unsubstituiertes Aryloxy oder Arylthio, oder für Acyloxy oder Silyloxy steht, $R_1$ für Hydroxy, Alkoxy oder Amino steht, und

$R_2$,$R_3$ gleich oder verschieden sein können und für Wasserstoff, geradkettige oder verzweigte, gegebenenfalls durch Halogen oder Phenyl substituiertes $C_1$-$C_8$-Alkyl-, $C_2$-$C_4$-Alkenyl-, und Alkinyl-, $C_1$-$C_8$-Hydroxyalkyl-, Alkoxyalkyl-, Aminoalkyl-, Cycloalkyl- oder Aralkyl-Reste, für einen aliphatischen oder aromatischen Acylrest oder für einen Silylrest, oder für substituiertes oder unsubstituiertes Aryl, wobei bevorzugte Substituenten des Arylrestes beispielsweise Halogen, Alkyl, Alkoxy, Halogenalkyl, Halogenalkoxy sowie substituiertes oder unsubstituiertes Aryloxy oder Arylthio sind, oder für bestimmte

Heterocyclen wie für Imidazolinyl, Thiazolinyl oder Pyrimidyl stehen, oder zusammen mit dem Stickstoffatom einen gegebenenfalls substituierten Pyrimidin-, Piperidin-, Piperazin-, Morpholin- oder einen gesättigten bicyclischen Rest bilden, und

$R_4$ für Wasserstoff, einen aliphatischen oder aromatischen Acylrest oder einen Silylrest steht,

und deren physiologisch verträglichen Salze.

2) Phenylethylamin-Derivate nach Anspruch 1, in denen

X,Y und Z gleich oder verschieden sein können und Wasserstoff, Halogen, Alkyl, Hydroxy oder Alkoxy bedeuten,

$R_1$ Wasserstoff, Hydroxy, $C_1$-$C_4$-Alkoxy oder substituiertes oder unsubstituiertes $C_6$-$C_{10}$-Aryloxy oder Arylthio bedeutet, und

$R_2$,$R_3$ gleich oder verschieden sein können und für Wasserstoff, gegebenenfalls durch Halogen, Hydroxy oder Phenyl substituierte niedere Alkylreste oder für gegebenenfalls durch Halogen, $C_1$-$C_4$-Alkyl-, $C_1$-$C_4$-Alkoxy-, $C_6$-$C_{10}$-Aryloxy- oder Arylthio-substituierte Phenylreste stehen, und

$R_4$ für Wasserstoff steht.

3) Phenylethylamin-Derivate nach Anspruch 1, in denen

X,Y und Z gleich oder verschieden sein können und für Wasserstoff oder Halogen stehen,

$R_1$ für Hydroxy oder $C_1$-$C_4$-Alkoxy steht, und

$R_2$,$R_3$ gleich oder verschieden sein können und Wasserstoff, $C_1$-$C_4$-Alkylreste oder durch gegebenenfalls substituiertes $C_6$-$C_{10}$-Aryloxy substituiertes Phenyl bedeuten, und

$R_4$ Wasserstoff bedeutet.

4) Verfahren zur Herstellung von Phenylethylamin-Derivaten der Formel (I) gemäß Anspruch 1, dadurch gekennzeichnet, daß man

a) Verbindungen der Formel (III)

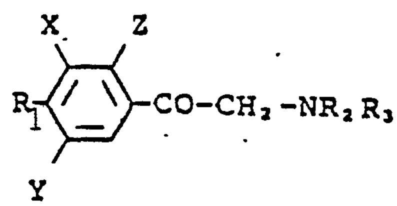 (III)

in der

X,Y,Z,$R_1$,$R_2$ und $R_3$ die oben angegebene Bedeutung haben,

reduziert oder daß man

b) Verbindungen der Formel (IV)

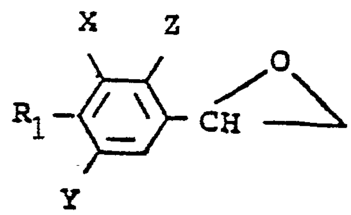

(IV)

in der
X,Y,Z und $R_1$ die oben angegebene Bedeutung haben,

mit Verbindungen der Formel (V)

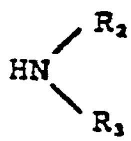

(V)

in der
$R_2$ und $R_3$ die oben angegebene Bedeutung haben,

umsetzt oder zur Herstellung von Verbindungen der Formel I, in denen $R_1$ Acyloxy oder Silyloxy ist und/oder $R_2$ bzw. $R_3$ und/oder $R_4$ für einen aliphatischen oder aromatischen Acyl- bzw. Silylrest stehen,

c) nach bekannten Methoden Verbindungen der allgemeinen Formel I, in der $R_1$ Hydroxy und/oder $R_2$ bzw. $R_3$ und/oder $R_4$ Wasserstoff bedeuten, mit geeigneten Mitteln acyliert oder silyliert.

5) Verfahren gemäß Anspruch 4,dadurch gekennzeichnet, daß man Verbindungen der Formel (III)

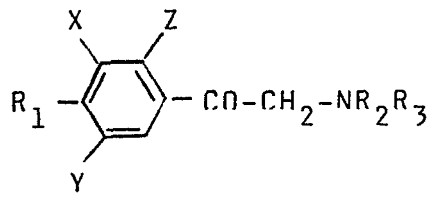

(III)

in der

X. Y und Z gleich oder verschieden sein können und Wasserstoff, Halogen, Alkyl, Hydroxy oder Alkoxy bedeuten,

$R_1$ Wasserstoff, Hydroxy, $C_1$-$C_4$-Alkoxy oder substituiertes oder unsubstituiertes $C_6$-$C_{10}$-Aryloxy oder Arylthio bedeutet und

$R_2$,$R_3$ gleich oder verschieden sein können und für Wasserstoff, gegebenenfalls durch Halogen, Hydroxy oder Phenyl substituierte niedere Alkylreste oder für gegebenenfalls Halogen, $C_1$-$C_4$-Alkyl, $C_1$-$C_4$-Alkoxy, $C_6$-$C_{10}$-Aryloxy oder Arylthio substituierte Phenylreste stehen,

mit Natriumborhydrid oder in Gegenwart eines Paladiumkatalysators hydriert.

6) Verfahren nach Anspruch 4, dadurch gekennzeichnet, daß man Verbindungen der Formel (IV)

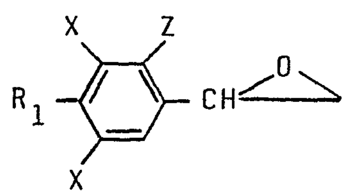

(IV)

in der

X, Y und Z gleich oder verschieden sein können und Wasserstoff, Halogen, Alkyl, Hydroxy oder Alkoxy bedeuten und

$R_1$ Wasserstoff, Hydroxy, $C_1$-$C_4$-Alkoxy oder substituiertes oder unsubstituiertes $C_6$-$C_{10}$-

Aryloxy oder Arylthio bedeutet,
mit Verbindungen der Formel (V)

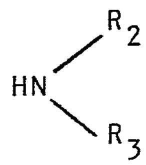

(V)

in der

$R_2$ und $R_3$ gleich oder verschieden sein können und für Wasserstoff, gegebenenfalls durch Halogen, Hydroxy oder Phenyl substituierte niedere Alkylreste oder für gegebenenfalls Halogen, $C_1$-$C_4$-Alkyl, $C_1$-$C_4$-Alkoxy, $C_6$-$C_{10}$-Aryloxy oder Arylthio substituierte Phenylreste stehen,

umsetzt.

7) Verwendung von Phenylethylamin-Derivaten der allgemeinen Formel (I)

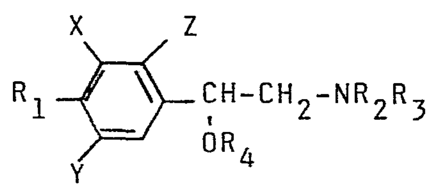

(I)

in der

X,Y und Z gleich oder verschieden sind und Wasserstoff, Halogen, Alkyl, Alkoxy, Alkenyl, Hydroxy, Cyano oder eine COR'-Gruppe bedeuten, und

$R_1$ für Wasserstoff, Alkyl, Halogen, Hydroxy, Alkoxy, Alkylthio, substituiertes oder unsubstituiertes Aryl, substituiertes oder unsubstituiertes Aryloxy oder Arylthio, oder für Acyloxy oder Silyloxy steht,

R' für Hydroxy, Alkoxy oder Amino steht, und

$R_2$,$R_3$ gleich oder verschieden sein können und für Wasserstoff, geradkettige oder verzweigte, gegebenenfalls durch Halogen oder Phenyl substituiertes $C_1$-$C_8$-Alkyl-, $C_2$-$C_4$-Alkenyl-, und Alkinyl-, $C_1$-$C_8$-Hydroxyalkyl, Alkoxyalkyl-, Aminoalkyl-, Cycloalkyl- oder Aralkyl-Reste, für einen aliphatischen oder aromatischen Acylrest oder einen Silylrest oder für substituiertes oder unsubstituiertes Aryl, wobei bevorzugte Substituenten des Arylrestes beispielsweise Halogen, Alkyl, Alkoxy, Halogenalkyl, Halogenalkoxy sowie substituiertes oder unsubstituiertes Aryloxy oder Arylthio sind, oder für bestimmte Heterocyclen wie für Imidazolinyl, Thiazolinyl

oder Pyrimidyl stehen, oder zusammen mit dem Stickstoffatom einen gegebenenfalls substituierten Pyrimidin-, Piperidin-, Piperazin-, Morpholin- oder einen gesättigten bicyclischen Rest bilden, und

$R_4$ Wasserstoff oder einen aliphatischen oder aromatischen Acylrest oder einen Silylrest bedeutet,

und deren physiologisch verträglichen Salze als Wachstumsförderer für Tiere.

8) Verwendung von Phenylethylamin-Derivaten der Formel (I), in der

X,Y und Z gleich oder verschieden sein können und Wasserstoff, Halogen, Alkyl, Hydroxy oder Alkoxy bedeuten,

$R_1$ Wasserstoff, Hydroxy, $C_1$-$C_4$-Alkoxy oder substituiertes oder unsubstituiertes $C_6$-$C_{10}$-Aryloxy oder Arylthio bedeutet, und

$R_2$,$R_3$ gleich oder verschieden sein können und für Wasserstoff, gegebenenfalls durch Halogen, Hydroxy oder Phenyl substituierte niedere Alkylreste oder für gegebenenfalls durch Halogen, $C_1$-$C_4$-Alkyl-, $C_1$-$C_4$-Alkoxy-, $C_6$-$C_{10}$-Aryloxy- oder Arylthio-substituierte Phenylreste stehen, und

$R_4$ Wasserstoff bedeutet,

als Wachstumsförderer für Tiere.

9) Verwendung von Phenylethylamin-Derivaten der Formel (I), in der

X,Y und Z gleich oder verschieden sein können und für Wasserstoff oder Halogen stehen,

$R_1$ für Hydroxy oder $C_1$-$C_4$-Alkoxy steht, und

$R_2$,$R_3$ gleich oder verschieden sein können und Wasserstoff, $C_1$-$C_4$-Alkylreste oder durch gegebenenfalls substituiertes $C_6$-$C_{10}$-Aryloxy substituiertes Phenyl bedeuten, und

$R_4$ Wasserstoff bedeutet,

als Wachstumsförderer für Tiere.

10) Verwendung von Phenylethylamin- Derivaten der allgemeinen Formel II

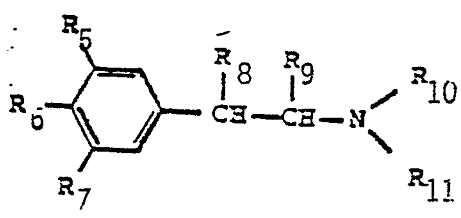

(II)

in welcher

$R_5$, $R_6$ und $R_7$ gleich oder verschieden sind und jeweils für Wasserstoff, Hydroxy, Alkoxy oder Hydroxymethyl stehen, und

$R_8$ Wasserstoff oder Hydroxy bedeutet und

$R_9$ für Wasserstoff, geradkettiges, verzweigtes oder cyclisches Alkyl oder Alkenyl, Aryl, Acyl oder Aroyl steht, wobei die genannten Alkyl-, Alkenyl- und Arylreste gegebenenfalls substituiert sind durch Halogen, Hydroxy, Alkyl, Alkoxy, Amino, gegebenenfalls substituiertes Phenyl oder Heteroaryl, und

$R_{10}$ und $R_{11}$ gleich oder verschieden sind und jeweils für Wasserstoff, geradkettiges, verzweigtes oder cyclisches Alkyl, Alkenyl, Aryl, Acyl, Aroyl, Mono- und Dialkylaminoalkyl, Alkoxyalkyl, Phenoxyalkyl oder Acylamino stehen, wobei die genannten Alkyl, Alkenyl- und Arylreste gegebenenfalls substituiert sind durch Halogen, Amino, Alkyl, Alkoxy, Hydroxy, Acylamino, gegebenenfalls substituiertes Phenyl oder Heteroaryl, oder für

$R_{10}$ und $R_{11}$ gemeinsam mit dem Stickstoffatom einen gegebenenfalls substituierten Pyrrolidin-, Piperidin-, Piperazin- oder Morpholin-Rest bilden,

und deren physiologisch verträglichen Salze als Wachstumsförderer für Tiere.

11) Verwendung von Phenylethylamin-Derivaten der allgemeinen Formel II gemäß Anspruch 8, in welcher

$R_5$, $R_6$ und $R_7$ gleich oder verschieden sind und jeweils für Wasserstoff, Hydroxy, Hydroxymethyl oder Methoxy stehen,

$R_8$ Wasserstoff oder Hydroxy bedeutet,

$R_9$ für Wasserstoff, geradkettige oder verzweigte Alkyl- oder Alkenyl-Gruppen mit bis zu 5 Kohlenstoffatomen, Phenyl oder Aralkyl mit 7 bis 10 Kohlenstoffatomen steht, wobei die genannten Aryl-Reste gegebenenfalls substituiert sind durch Chlor, Fluor, niederes Alkyl bis zu 4 Kohlenstoffatomen, Hydroxy oder Alkoxy, und

$R_{10}$ und $R_{11}$ gleich oder verschieden sein können und für Wasserstoff, geradkettiges oder verzweigtes Alkyl mit 1 bis 8 Kohlenstoffatomen, Alkenyl mit 2 bis 4 Kohlenstoffatomen, Hydroxyalkyl, Mono- und Dialkylaminoalkyl und Phenylalkylaminoalkyl mit jeweils bis zu 4 Kohlenstoffatomen in den Alkylresten, oder Phenyl-, Aralkyl-, Methylendioxyphenylalkyl-, Alkoxyalkyl-Reste mit 8 bis 12 Kohlenstoffatomen oder Phenoxyalkylreste mit 8 bis 16 Kohlenstoffatomen, wobei die genannten Alkylgruppen gegebenenfalls durch Methyl oder Ethyl und die Phenylreste gegebenenfalls durch Halogen, insbesondere durch Chlor oder Fluor, Hydroxy, niederes Alkyl oder niederes Alkoxy mit bis zu 2 Kohlenstoffatomen substituiert sein kann, stehen, oder

$R_{10}$ und $R_{11}$ gemeinsam mit dem Stickstoffatom einen gegebenenfalls substituierten Pyrrolidin-, Piperidin-, Piperazin- oder Morpholin-Rest bilden

als Wachstumsförderer für Tiere.

12) Verwendung von Phenylethylamin-Derivaten der allgemeinen Formel II gemäß Anspruch 8, in welcher

$R_5$, $R_6$ und $R_7$ gleich oder verschieden sind und jeweils für Wasserstoff oder Hydroxy stehen und

$R_8$ Hydroxy bedeutet, und

$R_9$ für Wasserstoff oder Methyl steht und

$R_{10}$ und $R_{11}$ gleich oder verschieden sind und jeweils für Wasserstoff, geradkettiges oder verzweigtes Alkyl mit jeweils bis zu 4 Kohlenstoffatomen stehen, wobei die Alkylgruppen gegebenenfalls durch Phenyl, Phenoxy, Hydroxyphenyl oder Methylendioxyphenyl substituiert sein können,

als Wachstumsförderer für Tiere.

13) Verwendung von Phenylethylamin-Derivaten der allgemeinen Formeln (I) oder (II) von Anspruch 1 bzw. 8 als wachstumsfördernder Zusatz in der Tiernahrung.

14) Verwendung von Phenylethylamin-Derivaten der allgemeinen Formeln (I) oder (II) von Anspruch 1 bzw. 8 gemäß einem oder mehreren der Ansprüche 1 bis 3 in Mengen von 0,01 bis 50 mg pro kg Körpergewicht pro Tag als Wachstumsförderer.

15) Tiernahrung enthaltend Phenylethylamin-Derivate der allgemeinen Formeln (I) oder (II) von Anspruch 1 bzw. 8.

16) Wachstumsförderndes Mittel für Tiere, bestehend aus Phenylethylamin-Derivaten der allgemeinen Formeln (I) oder (II) von Anspruch 1 bzw. 8.

17) Wachstumsförderndes Mittel für Tiere, enthaltend Phenylethylamin-Derivate der allgemeinen Formeln (I) oder (II) von Anspruch 1 bzw. 8.

18) Verfahren zur Herstellung von wachstumsfördernder Tiernahrung, dadurch gekennzeichnet, daß man der Tiernahrung Phenylethylamin-Derivate der allgemeinen Formeln (I) oder (II) von Anspruch 1 bzw. 8 zusetzt.

19) Praemixe für die Tiernahrung enthaltend Phenylethylamin-Derivate der allgemeinen Formeln (I) oder (II) von Anspruch 1 bzw. 8.